# EUROPEAN PATENT SPECIFICATION

(11) **EP 2 773 361 B1**
(45) Date of publication and mention of the grant of the patent: **27.02.2019**
(21) Application number: 12783362.2
(22) Date of filing: 30.10.2012
(51) Int. Cl.: A61K 31/7072, A61K 45/00, A61P 25/00, A61K 31/202, A61K 31/14, A61K 31/355, A61K 31/375, A61K 31/4415, A61K 31/519, A61K 31/675, A61K 31/683, A61K 31/685, A61K 31/7068, A61K 31/714, A61K 33/04, A23L 33/00, A23L 33/12, A23L 33/13, A61K 45/06

(54) **METHOD FOR IMPROVING EXECUTIVE FUNCTION**
METHODE ZUR VERBESSERUNG DER EXEKUTIVEN FUNKTION
MÉTHODE POUR AMÉLIORER LA FONCTION EXÉCUTIVE

(30) Priority: 31.10.2011 WO PCT/NL2011/050738
(43) Date of publication of application: 10.09.2014
(73) Proprietor: N.V. Nutricia, 2712 HM Zoetermeer (NL)
(72) Inventor: GROENENDIJK, Martine, NL-2992 GP Barendrecht (NL); BONGERS, Anke, NL-3906 BD Veenendaal (NL)
(74) Representative: Nederlandsch Octrooibureau
(86) International application number: PCT/NL2012/050753
(87) International publication number: WO 2013/066167

(56) References cited:
- WO-A1-2009/002146
- WO-A1-2009/002148

## Description

### Field of the invention

The invention is in the field of medical nutrition and more particularly relates to a composition for use in improving executive functions like speed of information processing, cognitive and mental flexibility, attention, scanning, and cognitive set shifting, in particular in a Alzheimer's or dementia patient.

### Background description

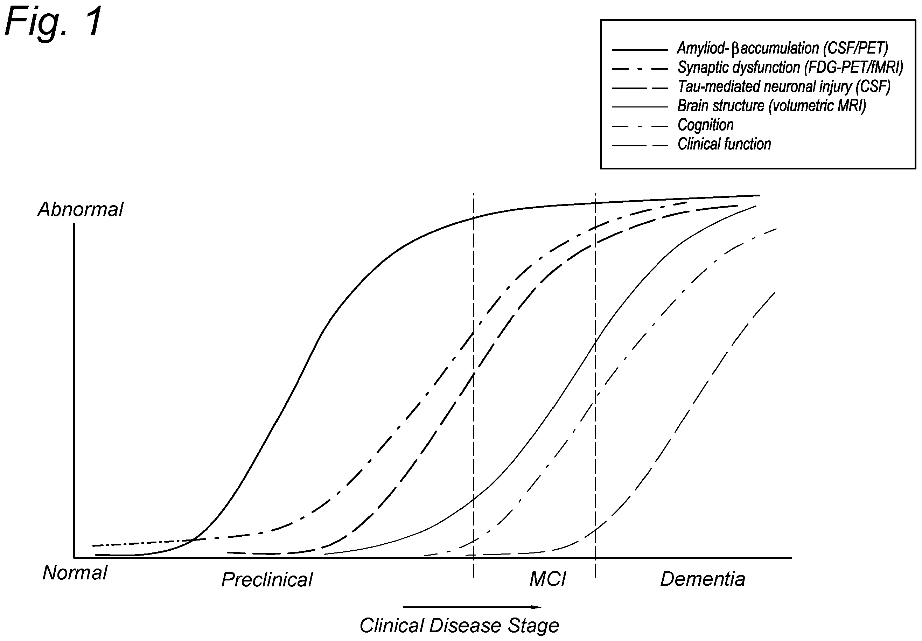

During the last decennium, uridine, choline and omega-3 fatty acids such as DHA have attracted attention as active components in treating cognitive dysfunction and age-associated memory impairment (AAMI), see e.g. WO2007/089703 (Massachusetts Institute of Technology) and WO 2009/002165 (N.V. Nutricia). These compounds are rate-limiting precursors for membrane phosphatide synthesis. According to the above applications, by improving the membrane phosphatide synthesis, it is believed to improve cognitive or memory function. The clinical stages of age-associated memory impairment are schematically shown in figure 1.

WO 2009/002164 (N.V. Nutricia) teaches similarly, albeit directed to earlier onset of dementia, during the so called prodromal stages. Neurodegeneration in the neocortex was investigated there. However, memory impairment such as associated with AD has many other serious consequences, such as reduced quality of life, difficulties in performing the activities of daily living, behavioral problems, potentially resulting in hospitalization or institutionalization. WO'64 however focuses on memory function.

WO 2009/002148 (N.V. Nutricia) pertains to the use of a composition comprising (a) one or more omega-3 fatty acids selected from DHA, DPA and EPA, (b) uridine or its equivalent, and (c) a methyl donor, in the manufacture of a preparation for the preparation for the prevention or delay of the onset of dementia in a person having characteristics of a prodromal dementia patient.

WO 2009/002146 (N.V. Nutricia) relates to the use of a composition comprising (a) DHA and/or EPA, and (b) uridine or its equivalent, in the manufacture of a composition for supporting activities in daily living.

WO 2009/002163 (N.V. Nutricia) deals with the difficulties in performing the activities of daily living in persons suffering from AD. It focuses on the operational activities and the executive brain function, i.e. the instrumental and/or basic activities of daily living of AD patients. The tool used there was an inventory involving a questionnaire of 23 items. No test for executive function was reported.

The effect of a medical nutrition containing a combination of specific nutrients DHA/EPA, UMP, choline, phospholipids and vitamins B, C and E and selenium was investigated in a clinical trial with people with mild Alzheimer's disease. The coprimary outcome measures were the delayed verbal recall test of the Wechsler Memory Scale-revised (WMS-r), which is seen as a sensitive measure of episodic memory; impaired in the early stage of AD; and the I3-item modified Alzheimer's Disease Assessment Scale - cognitive subscale (ADAS-cog). The results are reported in Scheltens et al., "Efficacy of a medical food in mild Alzheimer's disease: A randomized controlled trial" Alzheimer's & Dementia 6 (2010), 1-10. Significant improvement in the delayed verbal recall task was noted. Similarly, WO 2009/002166 (N.V. Nutricia) describes a composition comprising (a) uridine or uridine phosphate; and (b) DHA and/or EPA for improving delayed recall function in a subject with a MMSE of 24-26, based on a (delayed) verbal memory task (derived from Wechsler Memory Scale-revised) on drug naive, very mild AD subjects. While the ADAS-cog is a powerful and respected tool in assessing trends particularly in studies of patients with moderate AD (MMSE less than 20), there are other tests such as the Neuropsychological Test Battery (NTB) available that focus on the assessment of executive functions such as planning, strategy and working memory, which may be more sensitive in mild AD. In the ADAS-cog test effects on such executive functions are relatively unattended.

WO 2011/011721 (Amazentis SA) discloses compositions and methods for protecting brain health in neurodegenerative disorders. In one embodiment, the composition is provided in an amount sufficient to enhance long-term memory in a patient by a statistically significant amount when assessed by a Rey Auditory and Verbal Learning Test (RAVLT).

In the art there is a need for improving executive functions for those who suffer from disorders that result in executive function decline, and those who are at risk of developing such disorders.

### Summary of the invention

The inventors have observed that after administration of a product comprising (i) one or more of uridine and cytidine, or salts, phosphates, acyl derivatives or esters thereof, and (ii) a lipid fraction comprising at least one of docosahexaenoic acid (22:6; DHA), eicosapentaenoic acid (20:5; EPA) and docosapentaenoic acid (22:5; DPA), or esters thereof, executive functions like speed of information processing, cognitive and mental flexibility, attention, scanning, and cognitive set shifting can be improved, in particular in a Alzheimer's or dementia patient.

This conclusion was the result of clinical trials involving many neuropsychological tests. Neuropsychological tests are specifically designed tasks used to measure a psychological function known to be linked to a particular brain structure or pathway. Tests are used for research into brain function and in a clinical setting for the diagnosis of deficits. They usually involve the systematic administration of clearly defined procedures in a formal environment, and they form a core component of the process of conducting neuropsychological assessment, along with personal, interpersonal and contextual factors (source: http://en.wikipedia.org/wiki/Neuropsychological_test).

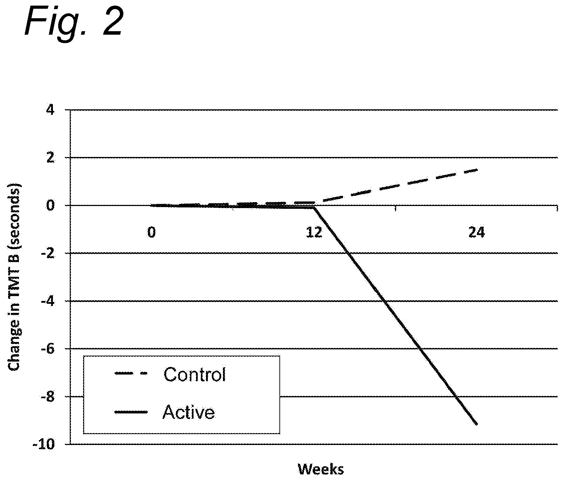

It was particularly surprising that the present clinical trials observations showed not merely a reduction in the rate of decline in executive functions in the patient group, but in fact an improvement. The results are plotted in figure 2. From tests for executive function commercially available, the results in terms of executive function presented herein were obtained with a so called Trail Making Test (abbreviated 'TMT'), part of a larger battery of neuropsychological tests. More details are provided here below.

### List of figures

**Figure 1** shows the different stages of cognitive decay in Alzheimer's Disease. Source: Sperling et al 2011;
**Figure 2** shows the results of a TMT B test (p=0.023; Mann-Whitney test). On the y-axis is the change in time needed to complete the complete test (in seconds), compared to the test as carried out at t = 0 weeks. A negative change means that the test is carried out faster than at t = 0 weeks;

### List of preferred embodiments

In one embodiment, the invention pertains to a composition for use in improving executive function of a subject in need thereof, wherein said composition comprises:
i) one or more of uridine and cytidine, or salts, phosphates, acyl derivatives or esters thereof; and
ii) a lipid fraction comprising at least one of docosahexaenoic acid (22:6; DHA), eicosapentaenoic acid (20:5; EPA) and docosapentaenoic acid (22:5; DPA), or esters thereof,
wherein said subject suffers from a memory or cognitive disorder, memory decline or cognitive dysfunction, such as Age Associated Memory Impairment (AAMI), Alzheimer's Disease, multiple sclerosis, vascular dementia, frontotemporal dementia, semantic dementia or dementia with Lewy bodies, and/or psychiatric and developmental disorders, including obsessive-compulsive disorder, Tourette's syndrome, depression, schizophrenia , attention-deficit/hyperactivity disorder, and autism (asperger).

In a further embodiment, the invention pertains to a composition for use treating a subject in need thereof, wherein said composition comprises:
i) one or more of uridine and cytidine, or salts, phosphates, acyl derivatives or esters thereof; and
ii) a lipid fraction comprising at least one of docosahexaenoic acid (22:6; DHA), eicosapentaenoic acid (20:5; EPA) and docosapentaenoic acid (22:5; DPA), or esters thereof,
and wherein said subject is subjected to a test for executive function,
wherein said subject suffers from a memory or cognitive disorder, memory decline or cognitive dysfunction, such as Age Associated Memory Impairment (AAMI), Alzheimer's Disease, multiple sclerosis, vascular dementia, frontotemporal dementia, semantic dementia or dementia with Lewy bodies, and/or psychiatric and developmental disorders, including obsessive-compulsive disorder, Tourette's syndrome, depression, schizophrenia , attention-deficit/hyperactivity disorder, and autism (asperger).

In the above embodiments, the test for executive function preferably comprises a trail making test [TMT], preferably a DKEFS TMT.

In the above embodiments, executive function preferably involves speed of information processing, cognitive and mental flexibility, attention, scanning, and/or cognitive set shifting,

In the above embodiments, said subject preferably suffers from Alzheimer's Disease or dementia syndrome, including mild or prodromal AD or dementia.

In the above embodiments, said subject preferably has a mini-mental state examination (MMSE) of 20 - 30, preferably 20 - 26, more preferably 24 - 26.

In the above embodiments, said composition preferably comprises choline, or salts or esters thereof, preferably 200 - 600 mg choline per daily dose or per 100 ml composition.

In the above embodiments, said composition preferably comprises at least one, preferably at least two, most preferably all B vitamins selected from the group consisting of vitamin B6, vitamin B12 and vitamin B9.

In the above embodiments, said composition preferably comprises, per daily dose or preferably per 100 ml composition, at least 500 mg of DHA, preferably at least 600 mg of DHA, and at least 50 mg of uridine, preferably at least 100 mg of uridine.

In the above embodiments, said composition preferably comprises, per daily dose or preferably per 100 ml composition:
50-1000 mg phospholipids,
0.5-3 mg vitamin B6,
50-500 □g folic acid,
1-30 □g vitamin B12.

In the above embodiments, said composition preferably comprises, per daily dose or preferably per 100 ml composition:
100 - 500 mg, preferably 200-400 mg EPA,
1000 - 1500 mg, preferably 1100-1300 mg DHA,
50 - 600 mg, preferably 60-200 mg phospholipids,
200 - 600 mg, preferably 300-500 mg choline,
400 - 800 mg, preferably 500-700 mg UMP (uridine monophosphate),
20 - 60 mg, preferably 30-50 mg vitamin E (alpha-TE),
60 - 100 mg, preferably 70-90 mg vitamin C,
40 - 80 µg, preferably 50-70 µg selenium,
1 - 5 µg, preferably 2-4 µg vitamin B12,
0.5 - 3 mg, preferably 0.5-2 mg vitamin B6, and
200 - 600 µg, preferably 300-500 µg folic acid.

### Detailed description of the invention

In one aspect, the invention pertains to a composition for use in improving executive functions of a subject in need thereof, wherein said composition comprises:
i) one or more of uridine and cytidine, or salts, phosphates, acyl derivatives or esters thereof; and
ii) a lipid fraction comprising at least one of docosahexaenoic acid (22:6; DHA), eicosapentaenoic acid (20:5; EPA) and docosapentaenoic acid (22:5; DPA), or esters thereof,
wherein said subject suffers from a memory or cognitive disorder, memory decline or cognitive dysfunction, such as Age Associated Memory Impairment (AAMI), Alzheimer's Disease, multiple sclerosis, vascular dementia, frontotemporal dementia, semantic dementia or dementia with Lewy bodies, and/or psychiatric and developmental disorders, including obsessive-compulsive disorder, Tourette's syndrome, depression, schizophrenia , attention-deficit/hyperactivity disorder, and autism (asperger).
In a preferred embodiment, the composition further comprises iii) choline, or salts or esters thereof.

In a further aspect, the invention pertains to a composition comprising (i)-(ii) and optionally (iii) as defined above, for use in treating a subject in need thereof, and subjecting said subject to a test for executive function, wherein said subject suffers from a memory or cognitive disorder, memory decline or cognitive dysfunction, such as Age Associated Memory Impairment (AAMI), Alzheimer's Disease, multiple sclerosis, vascular dementia, frontotemporal dementia, semantic dementia or dementia with Lewy bodies, and/or psychiatric and developmental disorders, including obsessive-compulsive disorder, Tourette's syndrome, depression, schizophrenia , attention-deficit/hyperactivity disorder, and autism (asperger). The composition is preferably administered to said subject at least on daily basis. The use preferably involves monitoring said subject with a test for executive function during the treatment.

The use of the invention comprises administering the composition comprising the aforementioned ingredients, and as further outlined below, to a subject in need thereof. The prophylactic or preventive aspect includes reducing the risk of occurring of the disorders.

The treatment preferably involves daily administration of the product, preferably for at least 12 weeks. The product is preferably administered (daily) for at least 13 weeks, more preferably at least 14, 15, 16, 17, 18, 19, 20, 21, 22, 23, most preferably at least 24 weeks. With 'improvement' it is intended to mean that the executive functions such as information processing and mental and cognitive flexibility increase compared to a control group of subjects suffering from the same condition but not given the composition of the invention.

### Executive function

The term 'executive function' describes a set of cognitive abilities that control and regulate other abilities and behaviors. Executive functions are high-level abilities that influence more basic abilities like attention, memory and motor skills. The executive functions are necessary for goal-directed behavior, and include the ability to initiate and stop actions, to monitor and change behavior as needed, and to plan future behavior when faced with novel tasks and situations. Executive functions allow us to anticipate outcomes and adapt to changing situations. The ability to form concepts and the ability to think abstractly are often considered components of executive function.

Executive functions are important for successful adaptation and performance in real-life situations. They allow people to initiate and complete tasks and to persevere in the face of challenges. Because the environment can be unpredictable, executive functions are vital to human ability to recognize the significance of unexpected situations and to make alternative plans quickly when unusual events arise and interfere with normal routines. In this way, executive function contributes to success in work and school and allows people to manage the stresses of daily life. Executive functions also enable people to inhibit inappropriate behaviors. People with poor executive functions often have problems interacting with other people since they may say or do things that are bizarre or offensive to others. Most people experience impulses to do or say things that could get them in trouble, such as making a sexually explicit comment to a stranger, commenting negatively on someone's appearance, or insulting an authority figure like a boss or police officer; but most people have no trouble suppressing these urges. When executive functions are impaired, however, these urges may not be suppressed. Executive functions are thus an important component of the ability to fit in socially.

The term 'executive function' in the context of the invention particularly comprises speed of information processing, cognitive and mental flexibility, attention, scanning, and/or cognitive set shifting, Executive function preferably consists of speed of information processing, cognitive and mental flexibility, attention, scanning, and/or cognitive set shifting. The invention is particularly concerned at the improvement of speed of information processing, and cognitive and mental flexibility, most preferably executive function in the context of the invention consists of speed of information processing.

Many of the tests which are commercially available to measure other abilities, particularly those that look at more complex aspects of these abilities, can be used to evaluate executive functions. For example, a person with executive function deficits may perform well on tests of basic attention, such as those that simply ask the individual to look at a computer screen and respond when a particular shape appears, but such person can have trouble with tasks that require divided or alternating attention, such as giving a different response depending on the stimulus presented. Verbal fluency tests that ask people to say a number of words in a certain period of time can also reveal problems with executive function. One commonly used test asks individuals to name as many animals or as many words beginning with a particular letter as they can in one minute. A person with executive function deficits may find the animal naming task simple, but struggle to name words beginning with a particular letter, since this task requires people to organize concepts in a novel way. Executive functions also influence memory abilities by allowing people to employ strategies that can help them remember information. Other tests are designed to assess cognitive function more directly. 'Tests for executive function' are acknowledged and standardized in the field. These tests are a tool to assess the above-explained particular aspects of memory performance, but are not a diagnosis. Such tests for executive function may present a fairly simple task but without instructions on how to complete it. Executive functions allow most people to figure out the task demanded through trial and error and change strategies as needed.

In the experimental part of the application, the subjects were tested for executive function deficits using a Trail Making Test B, or 'TMT-B'. The TMT was originally developed as part of the Army Individual Test Battery, and is one of the widely used neuropsychological tests for evaluating executive function. The TMT provides information on visual search, scanning, speed of processing, mental flexibility and other executive functions. Briefly, the TMT consists of two parts: TMT-A ('number sequencing') requires an individual to draw lines sequentially connecting a number of encircled numbers distributed on a sheet of paper. Task requirements are similar for TMT-B ('number - letter switching') except that the person must alternate between numbers and letters. The score on each part represents the amount of time to complete the task. More details are provided in T. Tombaugh "Trail Making Test A and B: Normative data stratified by age and education" Archives of Clinical Neuropsychology 19(2004) 203 - 214. It has also been addressed as a useful additive to the original Neuropsychological Test Battery [NTB] test as presented in J. Harrison et al. "10 Years of the Neuropsychological Test Battery (NTB)" Patient Reported Outcomes Newsletter 46 (Fall issue 2011) 21 - 24. More details are provided in Harrison et al. ARCH NEUROL/VOL 64 (NO. 9), SEP 2007, its contents considered herein incorporated by reference. The TMT test applied by the inventors was the Delis Kaplan Executive Function System™ (DKEFS; copyright © 2001 NCS Pearson, Inc.), particularly DKEFS condition 2 and 4 (corresponding to TMT A and B, respectively). The sequence of circles in the study was 16. For reasons stated above, the invention is however not considered limited to the particular test used by the inventors.

With the TMT-B (more particularly the DKEFS condition 4) as applied in the present case has been acknowledged a reliable means for test executive function, particularly speed of processing and cognitive flexibility, most preferably speed of processing. Part B of the Trail Making Test is a good general indicator because its cognitive demands include visual scanning, visual-motor coordination and visual-spatial ability adequate enough to understand on an ongoing basis the alternating pattern of numbers and letters. Part B is associated with the processes of distinguishing between numbers and letters, integration of two independent series, ability to learn an organizing principle and apply it systematically, serial retention and integration, verbal problem solving, and planning.

Based on foregoing, in a preferred embodiment the test for executive function comprises a TMT, preferably a DKEFS test. Most preferably, the test for executive function comprises a TMT B or DKEFS condition 4 test. Such test may be part of a NTB or other Battery of tests. In one aspect, the TMT is part of a Battery comprising Based on foregoing, in a preferred embodiment the test for executive function comprises a TMT, preferably a DKEFS test. Most preferably, the test for executive function comprises a TMT B or DKEFS condition 4 test. Such test may be part of a NTB or other Battery of tests. In one aspect, the TMT is part of a Battery comprising: (a) Wechsler memory scale [WMS] - verbal paired associates test; (b) Recognition test, preferably selected from the group consisting of: (i) Rey Auditory Verbal Learning test [RAVLT], (ii) Repeatable Battery for the Assessment of Neuropsychological Status [RBANS]; and (iii) California Verbal Learning Test [CVLT]; (c) WMS digit span test; (d) Controlled Word Association test [COWAT]; (e) Category fluency test; (f) Trail Making Test [TMT]; (g) Orientation task ADAS-cog test; and (h) Letter digit substitution test.

### Subject

The subject is a human being that suffers from a memory or cognitive disorder, memory decline or cognitive dysfunction, such as Age Associated Memory Impairment (AAMI), multiple sclerosis, vascular dementia, frontotemporal dementia, semantic dementia or dementia with Lewy bodies, and Alzheimer's Disease, and/or psychiatric and developmental disorders, including obsessive-compulsive disorder, Tourette's syndrome, depression, schizophrenia , attention-deficit/hyperactivity disorder, and autism (asperger). In the aforementioned conditions, executive functions are known to deteriorate.

The subject is a human being that suffers from a memory or cognitive disorder, memory decline or cognitive dysfunction. The subject is preferably suffering from cognitive dysfunction associated with Alzheimer's disease [AD], Pick's disease, Lewy Body disease, Huntington's disease, or 'dementia syndrome'. Dementia syndrome encompasses vascular dementia, frontotemporal dementia, semantic dementia.

The subject is a human, preferably an elderly human being, preferably at least 50 years of age. The subject is preferably an AD or dementia patient. In one aspect, the invention is not concerned with the treatment of Alzheimer's disease or dementia itself, but with the treatment of persons suffering from Alzheimer's disease, dementia and/or elderly.

In one embodiment, the subject is preferably a drug-naïve subject, which subject has preferably not been administered any drug for memory improvement and or for AD or dementia at least 4 weeks prior to the administration of a composition according to the invention. Preferably, the term 'drug naïve' as used in the present invention refers to subjects who do not ingest one or more of cholinesterase inhibitors, N-methyl-D-aspartate (NMDA) antagonists and ginkgo biloba during treatment with the composition of the invention, and preferably have not taken any cognitive ability-affecting drugs in the 4 weeks prior to the treatment.

The patient group subject in the clinical trials suffered from mild Alzheimer's disease. Hence, in one aspect, the subject is a mild cognitive impairment (MCI) patient (or 'mild AD patient' or 'mild dementia patient') or an AAMI patient. The patient group may also encompass prodromal patients of neurological disorders, in particular prodromal AD patients or drug-naïve prodromal dementia patients. A 'prodromal dementia patient' is a person who does not suffer from a senile dementia as defined above, but has an increased likelihood to develop senile dementia. Likewise a 'prodromal Alzheimer patient' is a person who does not suffer from AD, but has an increased likelihood to develop AD. The diagnostic tools that are used to classify the patients as prodromal patients are available in the art, and for instance summarized in WO 2009/002164, its contents herein incorporated by reference.

In yet a further way of characterizing the subject may be characterized by having a mini-mental state examination [MMSE] of 20 - 30. The MMSE is a standardized test developed in the art to distinguish between the various (pre-) stages of dementia. It involves a brief 30-point questionnaire that is used to assess cognition. In the time span of about 10 minutes it samples various functions including memory and orientation. The MMSE test includes simple questions and problems in a number of areas: the time and place of the test, repeating lists of words, language use and comprehension, and basic motor skills. Any score of 27 or higher (out of 30) is interpreted as effectively normal; 20-26 indicates mild dementia; 10-19 moderate dementia, and below 10 severe dementia. Copyrights prevent the inventors from including a copy of the questionnaire into the specification, but it is readily accessible on the internet and available through copyright owner Psychological Assessment Resources (PAR). It is first introduced by Folstein et al. (Psych Res 12:189, 1975), and is widely used with small modifications to assess cognition. Preferably, in the present invention, the subjects have a mini-mental state examination (MMSE) of 20-30, more preferably of 20-26, even more preferably a MMSE of 24, 25 or 26. More preferably, the subject having the aforementioned MMSE score range has (or suffers from) Alzheimer's disease, mild cognitive impairment (MCI), age-associated memory impairment (AAMI), multiple sclerosis, vascular dementia, frontotemporal dementia, semantic dementia or dementia with Lewy bodies.

Most preferably, the subjects as treated in the present invention suffer from mild Alzheimer's disease characterized by a MMSE of 20-26, preferably 24 - 26. In one embodiment, the subject is drug naïve.

### Product

Throughout the application, the terms 'product' and 'composition' are used interchangeably and account for the combination of ingredients administered to a subject in need thereof.

In one aspect of the present invention, the composition according to the invention may be used as a pharmaceutical product comprising one or more pharmaceutically acceptable carrier materials.

In another aspect of the present invention, the composition according to the invention may be used as a nutritional product, for example as a nutritional supplement, e.g., as an additive to a normal diet, as a fortifier, to add to a normal diet, or as a complete nutrition.

The pharmaceutical product, preferably for enteral application, may be a solid or liquid galenical formulation. Examples of solid galenical formulations are tablets, capsules (e.g. hard or soft shell gelatine capsules), pills, sachets, powders, granules and the like which contain the active ingredient together with conventional galenical carriers. Any conventional carrier material can be utilized. The carrier material can be organic or inorganic inert carrier material suitable for oral administration. Suitable carriers include water, gelatine, gum Arabic, lactose, starch, magnesium stearate, talc, vegetable oils, and the like. Additionally, additives such as flavouring agents, preservatives, stabilizers, emulsifying agents, buffers and the like may be added in accordance with accepted practices of pharmaceutical compounding. While the individual active ingredients are suitably administered in a single composition, they may also be administered in individual dosage units.

If the composition is a pharmaceutical product, such product may contain the daily dosage in one or more dosage units. The dosage unit may be in a liquid form or in a solid form, wherein in the latter case the daily dosage may be provided by one or more solid dosage units, e.g. in one or more capsules or tablets.

In another aspect of the present invention, the composition according to the invention may be used in a nutritional product comprising at least one component selected from the group of fats, proteins, and carbohydrates. It is understood that a nutritional product differs from a pharmaceutical product by the presence of nutrients which provide nutrition to the subject to which the composition is administered, in particular the presence of protein, fat, digestible carbohydrates and dietary fibres. It may further contain ingredients such as minerals, vitamins, organic acids, and flavouring agents. Although the term "nutraceutical product" is often used in literature, it denotes a nutritional product with a pharmaceutical component or pharmaceutical purpose. Hence, the nutritional composition according to the invention may also be used in a nutraceutical product.

The product of the invention is an enteral composition, intended for oral administration. It is preferably administered in liquid form. In one embodiment, the product comprises a lipid fraction and at least one of carbohydrates and proteins, wherein the lipid composition provides between 20 and 50 energy % of the food product. In one embodiment, the food product is a liquid composition containing between 0.8 and 1.4 kcal per ml.

Preferably, the composition comprising (i) and (ii) further comprises choline.

Preferably the composition comprising (i) and (ii) further comprises one or more of: phospholipids, vitamin E, vitamin C, selenium, vitamin B12, vitamin B6 and folic acid.

More preferably the composition comprises DHA, EPA, a uridine source (preferably UMP), phospholipids, choline, vitamin E, vitamin C, selenium, vitamin B12, vitamin B6 and folic acid.

### DHA/EPA

The composition comprises at least one ω-3 polyunsaturated fatty acid (LC PUFA; having a chain length of 18 and more carbon atoms) selected from the group consisting of docosahexaenoic acid (22:6; DHA), eicosapentaenoic acid (20:5; EPA) and docosapentaenoic acid (22:5 ω-3; DPA), preferably at least one of DHA and EPA. Preferably the present composition contains at least DHA, more preferably DHA and EPA. EPA is converted to DPA (ω-3), increasing subsequent conversion of DPA to DHA in the brain. Hence, the present composition preferably contains a significant amount of EPA, so to further stimulate in vivo DHA formation.

The DHA, EPA and/or DPA are preferably provided as triglycerides, diglycerides, monoglycerides, free fatty acids or their salts or esters, phospholipids, lysophospholipids, glycerol ethers, lipoproteins, ceramides, glycolipids or combinations thereof. Preferably, the present composition comprises at least DHA in triglyceride form.

In terms of daily dosage, the present use preferably comprises the administration of 400 to 5000 mg DHA+EPA+DPA (preferably DHA+EPA) per day, more preferably 500 to 3000 mg (preferably DHA+EPA) per day, most preferably 1000 to 2500 mg (preferably DHA+EPA) per day. DHA is preferably administered in an amount of 300 to 4000 mg per day, more preferably 500 to 2500 mg per day.

The present composition preferably comprises 1-40 wt.% DHA based on total fatty acids, preferably 3-36 wt.% DHA based on total fatty acids, more preferably 10-30 wt.% DHA based on total fatty acids. The present composition preferably comprises 0.5-20 wt.% EPA based on total fatty acids, preferably 2-10 wt.% EPA based on total fatty acids, more preferably 5-10wt.% EPA based on total fatty acids. The above-mentioned amounts take into account and optimise several aspects, including taste (e.g. too high LCP levels reduce taste, resulting in a reduced compliance).

The present composition preferably contains at least one oil selected from fish oil, algae oil and eggs lipids. Preferably the present composition contains fish oil comprising DHA and EPA.

The ratio of the weights of DHA to EPA is preferably larger than 1, more preferably 2:1 to 10:1, more preferably 3:1 to 8:1. The above-mentioned ratios and amounts take into account and optimise several aspects, including taste (too high LCP levels reduce taste, resulting in a reduced compliance), balance between DHA and precursors thereof to ensure optimal effectiveness while maintaining low-volume formulations.

Sources of DHA possible sources of DHA: tuna oil, (other) fish oils, DHA rich alkyl esters, algae oil, egg yolk, or phospholipids enriched with n-3 LCPUFA e.g. phosphatidylserine-DHA.

The present composition preferably contains a very low amount of arachidonic acid (AA). Preferably the weight ratio DHA/AA in the present composition is at least 5, preferably at least 10, more preferably at least 15, preferably up to e.g. 30 or even up to 60. The present use preferably comprises the administration of a composition comprising less than 5 wt.% arachidonic acid based on total fatty acids, more preferably below 2.5 wt.%, e.g. down to 0.5 wt%.

### ALA/LA

It is preferred that the alpha-linolenic acid [ALA] content of the composition is maintained at low levels. The ALA concentration may preferably be maintained at levels less than 2.0 weight%, more preferably below 1.5 weight%, particularly below 1.0 weight%, calculated on the weight of all fatty acids.

Linoleic acid [LA] concentrations can be maintained at normal levels, i.e. between 20 to 30 weight%, although in one embodiment the LA concentration is also significantly reduced to an amount of < 15 g/100 g fatty acids and even less than 10weight%. The LA concentrations are preferably at least 1 weight% of the fatty acids.

The weight ratio omega-6/omega-3 fatty acids in the present product is preferably below 0.5, more preferably below 0.2, e.g. down to 0.05 or to 0.01. The ratio ω-6/ω-3 fatty acids (C 20 and higher) in the present product is preferably below 0.3, more preferably below 0.15, e.g. down to 0.06 or to 0.03.

### MCT

In one embodiment, the composition contains less than 5 weight%, preferably less than 2 weight% of fatty acids of less than 14 carbon atoms.

Medium chain fatty acids [MCT] are defined to be linear or branched saturated carboxylic acids having six (C6:0), seven (C7:0), eight (C8:0), nine (C9:0) or ten (C10:0) carbon atoms. The amount of MCTs are preferably lower than 2 weight%, more preferably lower than 1.5 weight%, most preferably lower than 1.0 weight% of the total fatty acids. In one embodiment, the sum of the medium chain fatty acids C6:0 + C7:0 + C8:0 over the sum of C9:0 and C10:0 is less than 2:1, more preferably less than 1.8:1, most preferably less than 1.6:1.

### Saturated and monounsaturated fatty acids

The present composition preferably comprises saturated and/or monounsaturated fatty acids. The amount of saturated fatty acids is preferably 6-60 wt.% based on total fatty acids, preferably 12-40 wt.%, more preferably 20-40 wt.% based on total fatty acids. In particular the amount of C14:0 (myristic acid) + C16:0 (palmitic acid) is preferably 5-50 wt.%, preferably 8-36 wt.%, more preferably 15-30 wt.%, based on total fatty acids. The total amount of monounsaturated fatty acids, such as oleic acid and palmitoleic acid, is preferably between 5 and 40 wt.%, more preferably between 15 and 30 wt.%. A composition with these preferred amounts was found to be very effective.

### Uridine, UMP

The present composition comprises uridine, cytidine and/or an equivalent thereof, including salts, phosphates, acyl derivatives and/or esters. In terms of uridine, the composition preferably comprises at least one uridine or an equivalent thereof selected from the group consisting of uridine (i.e. ribosyl uracil), deoxyuridine (deoxyribosyl uracil), uridine phosphates (UMP, dUMP, UDP, UTP), nucleobase uracil and acylated uridine derivatives. In one embodiment, cytidine, CMP, citicoline (CDP-choline) may also be applied. Preferably, the composition to be administered according to the present invention comprises a source of uridine selected from the group consisting of uridine, deoxyuridine, uridine phosphates, uracil, and acylated uridine, and cytidine, more preferably selected from the group consisting of uridine, deoxyuridine, uridine phosphates, uracil, and acylated uridine.

Preferably, the present composition comprises an uridine phosphate selected from the group consisting of uridine monophosphate (UMP), uridine diphosphate (UDP) and uridine triphosphate (UTP); and/or a cytidine phosphate (CMP, CDP, CTP, preferably CMP). Most preferably the present composition comprises UMP, as UMP is most efficiently being taken up by the body. Preferably at least 50 weight% of the uridine in the present composition is provided by UMP, more preferably at least 75 weight%, most preferably at least 95 weight%. Doses that must be administered are given as UMP. The amount of uracil sources can be calculated taking the molar equivalent to the UMP amount (molecular weight 324 Dalton).

The present use preferably comprises the administration of uridine (the cumulative amount of uridine, deoxyuridine, uridine phosphates, nucleobase uracil and acylated uridine derivatives) in an amount of in an amount of 0.08-3 g per day, preferably 0.1-2 g per day, more preferably 0.2-1 g per day. The present use preferably comprises the administration of a composition comprising uridine in an amount of 0.08-3 g UMP per 100 ml liquid product, preferably 0.1-2 g UMP per 100 ml liquid product, more preferably 0.2-1 g per 100 ml liquid product. Preferably 1-37.5 mg UMP per kilogram body weight is administered per day. The above amounts also account for any amounts of cytidine, cytidine phosphates and citicoline incorporated in the composition or use.

Preferably, the present composition comprises uridine phosphate, preferably uridine monophosphate (UMP). The UMP is very efficiently taken up by the body. Hence, inclusion of UMP in the present composition enables a high effectivity at the lowest dosage and/or the administration of a low volume to the subject.

### Choline

In a preferred embodiment, the present composition contains choline, a choline salt and/or choline ester. For the remainder of the paragraph, the term 'choline' shall be considered to encompass all these equivalents. The choline salt is preferably selected from choline chloride, choline bitartrate, or choline stearate. The choline ester is preferably selected from a phosphatidylcholine and lysophosphatidyl choline. The present use preferably comprises the administration of more than 50 mg choline per day, preferably 80 to 2000 mg choline per day, more preferably 120 to 1000 mg choline per day, most preferably 150 to 600 mg choline per day. The present composition preferably comprises 50 mg to 3000 gram choline per 100 ml of the liquid composition, preferably 200 mg to 1000 mg choline per 100 ml. The above numbers are based on choline, the amounts of choline equivalents or sources can be calculated taking the molar equivalent to choline into account.

### Phospholipids

Preferably, the present composition preferably comprises phospholipids, preferably 0.1-50 wt.% phospholipids based on total weight of lipids, more preferably 0.5-20 wt.%, more preferably between 1 and 10% wt.%, most preferably between 1 and 5 wt.% based on total weight of lipids. The total amount of lipids is preferably between 10 and 30 wt.% on dry matter, and/or between 2 and 10 g lipid per 100 ml for a liquid composition. The composition preferably comprises between 0.01 and 1 gram lecithin per 100 ml, more preferably between 0.05 and 0.5 gram lecithin per 100 ml. A composition with these preferred amounts was found to be very effective.

### Vitamins

The present combination preferably comprises at least one B complex vitamin. The vitamin B is selected from the group of vitamin B1 (thiamine), vitamin B2 (riboflavin), vitamin B3 (niacin or niacinamide), vitamin B5 (pantothenic acid), vitamin B6 (pyridoxine, pyridoxal, or pyridoxamine, or pyridoxine hydrochloride), vitamin B7 (biotin), vitamin B9 (folic acid or folate), and vitamin B12 (various cobalamins). Functional equivalents are encompassed within these terms.

Preferably, at least one vitamin B is selected from the group of vitamin B6, vitamin B12 and vitamin B9. Preferably the present composition comprises at least two selected from the group consisting of vitamin B6, vitamin B12 and vitamin B9. In particular, good results have been achieved with a combination comprising vitamin B6, vitamin B12 and vitamin B9. Again, functional equivalents are encompassed within these terms.

The vitamin B is to be administered in an effective dose, which dose depends on the type of vitamin B used. As a rule of thumb, a suitable minimum or a maximum dose may be chosen based on known dietary recommendations, for instance as recommended by Institute of Medicine (IOM) of the U.S. National Academy of Sciences or by Scientific Committee on Food (a scientific committee of the EU), the information disclosed herein and optionally a limited amount of routine testing. A minimum dose may be based on the estimated average requirement (EAR), although a lower dose may already be effective. A maximum dose preferably does not exceed the tolerable upper intake levels (UL), as recommended by IOM.

If present in the nutritional composition or medicament, the vitamin B6 is usually present in an amount to provide a daily dosage in the range of 0.1 to 100 mg, in particular in the range of 0.5 to 25 mg, more in particular in the range of 0.5 to 5 mg. The present composition preferably comprises 0.1 to 100 mg vitamin B6 per 100 g (liquid) product, more preferably 0.5 to 5 mg vitamin B6 per 100 g (liquid) product, more preferably 0.5 to 5 mg vitamin B6 per 100 g (liquid) product.

If present in the nutritional composition or medicament, the vitamin B12 is usually present in an amount to provide a daily dosage in the range of 0.5 to 15 µg, in particular in the range of 1 to 10 µg, more in particular in the range of 1.5 to 5 µg. The present composition preferably comprises 0.5-15 µg vitamin B12 per 100 g (liquid) product, more preferably 1 to 10 µg vitamin B12 per 100 g (liquid) product, more preferably 1.5 to 5 µg vitamin B12 per 100 g (liquid) product. The term "vitamin B12" incorporates all cobalbumin equivalents known in the art.

Throughout the application, the terms 'folic acid', 'folate' and 'B9' are used interchangeably. If present in the nutritional composition or medicament, the vitamin B9 is usually present in an amount to provide a daily dosage in the range of 50 to 1000 µg, in particular in the range of 150 to 750 µg, more in particular in the range of 200 to 500 µg. The present composition preferably comprises 50 to 1000 µg folic acid per 100 g (liquid) product, more preferably 150 to 750 µg folic acid per 100 g (liquid) product, more preferably 200 to 500 µg folic acid per 100 g (liquid) product. Folates include folic acid, folinic acid, methylated, methenylated and formylated forms of folates, their salts or esters, as well as their derivatives with one or more glutamic acid, and all in either reduced or oxidized form.

### Vitamins C, E

Vitamin C, or a functional equivalent thereof, may be present in an amount to provide a daily dosage in the range of 20 to 2000 mg, in particular in the range of 30 to 500 mg, more in particular in the range of 75 to150 mg. In one embodiment, vitamin C , or a functional equivalent thereof, is present in an amount in the range of 20 to 2000 mg, in particular in the range of 30 to 500 mg, more in particular in the range of 75 to150 mg per 100 ml of the composition.

Tocopherol and/or an equivalent thereof (i.e. a compound having vitamin E activity) may be present in an amount to provide a daily dosage in the range of 10 to 300 mg, in particular in the range of 30 to 200 mg, more in particular in the range of 35 to100 mg, to prevent oxidative damage resulting from dietary PUFA. In one embodiment, tocopherol and/or equivalent is present in an amount in the range of 10 to 300 mg, in particular in the range of 30 to 200 mg, more in particular in the range of 35 to100 mg per 100 ml of the composition. The term "tocopherol and/or an equivalent thereof", and 'alpha-TE', as used in this description, comprises tocopherols, tocotrienols, pharmaceutical and/or nutritional acceptable derivatives thereof and any combination thereof. The above numbers are based on tocopherol equivalents, recognized in the art.

### Selenium

The present composition preferably contains selenium, because of its antioxidant activity. Preferably the present use provides the administration of a composition comprising 0.01 and 5 mg selenium per 100 ml liquid product, preferably 0.02 and 0.1 mg selenium per 100 ml liquid product. The amount of selenium administered per day is preferably more than 0.01 mg, more preferably 0.01 to 0.5 mg.

### Protein

Although the composition may further comprise proteinaceous material, it has been found that such component is not deemed necessary. In fact, it is thus possible to concentrate the actives in a low volume composition. Should a protein fraction be included, the protein fraction comprises intact proteins, peptides as may be obtained by hydrolyses of intact proteins and by syntheses, derivatives of peptides comprising more than 80 weight% amino acids. Nitrogen from nucleosides material and choline will not be calculated as being protein.

In one embodiment, it is preferred that the amount of taurine (including taurine salts) is less than 0.1 g, preferably less than 0.05 g per daily dose. Additionally or alternatively, it is preferred that the amount of taurine (including taurine salts) is less than 5 mg, more preferably less than 2.5 g per 100 g composition.

In one embodiment, the composition comprises less than 25 mg, more preferably less than 20 mg, most preferably less than 15 mg cysteine and taurine per 100 ml of the (liquid) composition. In one embodiment, the composition comprises less than 25 mg, more preferably less than 20 mg, most preferably less than 15 mg cysteine per 100 ml of the (liquid) composition. It is preferred that the protein fraction comprises more than 70 weight% of casein or caseinates, or hydolysates thereof, and more preferably 80 weight% or more, because caseins comprise relatively low amounts of cysteine compared to other protein sources. It is further preferred to heat the liquid composition in order to oxidize the cysteine molecules present in the protein. This impairs biological availability of any residual cysteine as present in the formula. A preferred heat treatment involves sterilization. It is preferred to maintain the temperature remains below 135 °C, preferably less than 132 °C combined with a sufficient long time to have the cysteine oxidized, i.e. more than 30 seconds, preferably more than 40 seconds.

In one embodiment, it is preferred that the composition has a protein content of less than 15 en%, more preferably less than 10 en%, most preferably less than 5 en% of the total energy content of the composition. The energy percentages of the components are calculated using the calculation factors 9 kcal per g lipid, 4 kcal per g protein or g digestible carbohydrates, 2 kcal per g dietary fibers and zero kcal for the other components in the composition. In one embodiment, it is preferred that the composition comprises less than 0.5 to 10 g protein per 100 ml, more preferably less than 1 to 6 gram protein per 100 ml, most preferably 2 to 6 gram protein/100 ml.

A preferred composition according to the invention comprises, per daily dose or per 100 ml composition:
100 - 500 mg, preferably 200-400 mg EPA,
900 - 1500 mg, preferably 950-1300 mg DHA,
50 - 600 mg, preferably 60-200 mg phospholipids,
200 - 600 mg, preferably 300-500 mg choline,
400 - 800 mg, preferably 500-700 mg UMP (uridine monophosphate),
20 - 60 mg, preferably 30-50 mg vitamin E (alpha-TE),
60 - 100 mg, preferably 60-90 mg vitamin C,
40 - 80 µg, preferably 45-65 µg selenium,
1 - 5 µg, preferably 2-4 µg vitamin B12,
0.5 - 3 mg, preferably 0.5-2 mg vitamin B6, and
200 - 600 µg, preferably 300-500 µg folic acid.

More preferred, a composition according to the invention comprises per 100 ml composition:
100 - 500 mg, preferably 200-400 mg EPA,
900 - 1500 mg, preferably 950-1300 mg DHA,
50 - 600 mg, preferably 60-200 mg phospholipids,
200 - 600 mg, preferably 300-500 mg choline,
400 - 800 mg, preferably 500-700 mg UMP (uridine monophosphate),
20 - 60 mg, preferably 30-50 mg vitamin E (alpha-TE),
60 - 100 mg, preferably 60-90 mg vitamin C,
40 - 80 µg, preferably 45-65 µg selenium,
1 - 5 µg, preferably 2-4 µg vitamin B12,
0.5 - 3 mg, preferably 0.5-2 mg vitamin B6, and
200 - 600 µg, preferably 300-500 µg folic acid.

The compositions as described above can be used as a nutritional therapy, nutritional support, as a medical food, as a food for special medical purposes or as a nutritional supplement. Such product can be consumed at one, two or three servings between 75 and 200 ml per day or per unit, most preferably between 90 and 150 ml/day , most preferably about 125 mL per day in the aforementioned applications.

The subjects that can benefit from the composition for use of the invention often experience problems with eating. Their sensory capabilities and/or control of muscles can become imparted, as well as in some instances their ambition to apply proper eating habits. Swallowing and/or mastication may be problematic.

Hence, the present composition is preferably provided in the form of a drink capable of being ingested through a straw.

Related therewith, the composition according to the invention preferably has a low viscosity, preferably a viscosity between 1 and 2000 mPa.s measured at a shear rate of 100 sec-1 at 20 °C, more preferably a viscosity between 1 and 100 mPa.s measured at a shear rate of 100 sec-1 at 20 °C. In a preferred embodiment the present composition has a viscosity of 1- 80 mPas at a shear rate of 100 per sec at 20 °C, more preferably of 1-40 mPas at a shear rate of 100 per sec at 20 °C. These viscosity measurements may for instance be performed using plate and cone geometry.

To be optimally accepted by the subject, the present composition preferably has an osmolality of 300 to 800 mOsm/kg. However, the energy density of the product is preferably not so high that it interferes with normal eating habits. When in liquid form, the present product preferably contains between 0.2 and 3 kcal/ml, more preferably between 0.5 and 2, between 0.7 and 1.5 kcal/ml.

In a further aspect, the invention pertains to a composition for use in for improving executive function of a subject in need thereof, wherein said composition comprises (i)- (ii), and as further characterized here above; and monitoring said subject to a test for executive function, such as a Trail Making Test B, and wherein said subject suffers from a memory or cognitive disorder, memory decline or cognitive dysfunction, such as Age Associated Memory Impairment (AAMI), Alzheimer's Disease, multiple sclerosis, vascular dementia, frontotemporal dementia, semantic dementia or dementia with Lewy bodies, and/or psychiatric and developmental disorders, including obsessive-compulsive disorder, Tourette's syndrome, depression, schizophrenia , attention-deficit/hyperactivity disorder, and autism (asperger) The composition is particularly for use in improving speed of information processing and cognitive and mental flexibility, most preferably for use in improving speed of information processing.

### EXAMPLES

### Example 1: Packaged composition for comprising per 125 ml:

Energy 125 kcal; Protein 3.9 g; Carbohydrate 16.5 g; Fat 4.9 g.

Fat includes 1.5 g DHA + EPA, and 106 mg phospholipids (soy lecithin); Choline 400 mg; UMP (uridine monophosphate) 625 mg; Vitamin E 40 mg alpha-TE; Vitamin C 80 mg; Selenium 60 □g; Vitamin B12 3 □g; Vitamin B6 1 mg; Folic acid 400 □g.

Minerals and trace elements: Sodium 125 mg; Potassium 187.5 mg; Chloride 156.3 mg; Calcium 100 mg; Phosphorus 87.5 mg; Magnesium 25 mg; Iron 2 mg; Zinc 1.5 mg; Copper 225 µg; Manganese 0.41 mg; Molybdenum 12.5 µg; Chromium 8.4 µg; Iodine 16.3 µg. Vitamins: Vit. A 200 µg-RE; vit. D3 0.9 µg; vit. K 6.6 µg; Thiamin (B1) 0.19 mg; Riboflavin (B2) 0.2 mg; Niacin (B3) 2.25 mg-NE; Pantothenic acid (B5) 0.66 mg; Biotin 5 µg.

### Example 2. Clinical study

A proof-of-concept study in drug-naïve patients with mild AD (MMSE 20-26) showed that a composition according to the invention (see table 1; comprising DHA, EPA, UMP, choline, phospholipids, vitamins B6, B9, B12, vitamins C and E, Selenium) taken once per day was safe and improved delayed recall function of a subject after 12 weeks, the co-primary endpoint of the study. Source: Scheltens et al., "Efficacy of a medical food in mild Alzheimer'sd isease: A randomized controlled triat" Alzheimer's & Dementia 6 (2010), 1-10.

**Table 1. Nutritional composition used in clinical trials**

| component | Amount per daily dose |
|---|---|
| EPA | 300 mg |
| DHA | 1200 mg |
| Phospholipids | 106 mg |
| Choline | 400 mg |
| UMP | 625 mg |
| Vitamin E (alpha-TE) | 40 mg |
| Vitamin C | 80 mg |
| Selenium | 60 □g |
| Vitamin B12 | 3 □g |
| Vitamin B6 | 1 mg |
| Folic acid | 400 □g |

| | |
|---|---|
| *125 ml, daily dose. | |

The present study was designed to confirm the effect of the composition on memory in drug-naïve patients with mild AD, and also to extend the investigation through a longer intervention period of 24 weeks and through utilization of the whole memory domain z-score of a Neuropsychological Test Battery (NTB). Secondary objectives were to investigate safety and tolerance of the intervention, and to assess the effects on executive function (z score) (TMTs, CF, COWAT, digit span), total composite score NTB and individual items NTB.

### Material and methods

The study was a randomized, controlled, double-blind study, conducted at 27 study centers in six European countries (the Netherlands, Germany, France, Belgium, Italy and Spain). Drug-naïve patients with mild AD (MMSE scores ≥ 20) and diagnosis of probable AD according to the NINCDS-ADRDA criteria, were randomly assigned (1:1) to the composition including the components according to table 1, or an iso-caloric control product. The duration of intervention was 24 weeks.

The memory domain score of a Neuropsychological Test Battery (NTB) was the primary outcome parameter. This memory composite score was derived from the Rey Auditory Verbal Learning Test (RAVLT: immediate recall, delayed recall and recognition performance) and the Wechsler Memory Scale (WMS) verbal paired associates test (immediate and delayed recall).

Secondary outcomes resulting from the NTB were the executive function domain, total composite score and individual item scores. The other NTB items were WMS Digit Span, Trail Making Tests part A and B, Category Fluency, Controlled Word Association Test, the ADAS-cog orientation task and the Letter Digit Substitution Test. Other secondary outcome parameters were the Disability Assessment for Dementia (DAD) scale, EEG (basic frequency and functional connectivity analysis), product compliance, tolerance and safety. Main study parameters were assessed at baseline, week 12 and week 24. For the statistical analysis of the data, a repeated measures mixed model was used. The trial was registered with the ICMJE compliant www.trialregister.nl (NTR1975).

### Results

A total of 259 drug-naïve subjects were randomized (2.6% screen failures). In the overall study population no differences in baseline characteristics were noted between the study groups. The mean age was 73.8 (± 7.7) years, the mean screening MMSE was 25.0 (± 2.8) and 51% were male. A pre-specified blinded interim analysis was conducted to check whether the calculated sample size was adequate and that no safety concerns had arisen, and the independent Data Monitoring Committee recommended continuation of the trial without modification. From the 259 subjects randomized, 238 subjects (91.9%) completed the study. Five subjects did not complete the study because of a (serious) adverse event ((S)AE); 3 in the group having received the composition and 2 in the control group, and no differences between study groups were noted in the occurrence of (S)AEs. No clinically relevant differences in blood safety parameters were noted. The average compliance during 24 weeks was very high at 97% and not different between the groups. High compliance was confirmed by marked and significant changes in (nutritional) biomarkers of compliance, e.g. docosahexaenoic acid in erythrocyte membranes and plasma homocysteine.

During 24 weeks, the composition of the invention significantly improved the primary endpoint memory performance (composite memory domain z-score resulting from the NTB) compared to control product (repeated measures mixed model, p=0.025). The significant effect on memory performance was confirmed by individual tasks of the NTB memory domain.

Within the NTB executive function domain, an improvement was shown for the composition of the invention in the Trail Making Test B. The TMT B used was the so called DKEFS condition 4 [Delis Kaplan Executive Function System™. Copyright © 2001 NCS Pearson, Inc], There, the results in terms of executive function proved significant (see Figure 2). Details are provided in the text.

### Discussion

In conclusion, this study showed that 24-weeks of supplementation with the active composition improved EF as measured by the TMT B , and is well-tolerated in drug-naïve patients with mild AD.

## Claims

1. A composition for use in improving executive function of a subject in need thereof, wherein said composition comprises:
i) one or more of uridine and cytidine, or salts, phosphates, acyl derivatives or esters thereof; and
ii) a lipid fraction comprising at least one of docosahexaenoic acid (22:6; DHA), eicosapentaenoic acid (20:5; EPA) and docosapentaenoic acid (22:5; DPA), or esters thereof,
wherein said subject suffers from a memory or cognitive disorder, memory decline or cognitive dysfunction, such as Age Associated Memory Impairment (AAMI), Alzheimer's Disease, multiple sclerosis, vascular dementia, frontotemporal dementia, semantic dementia or dementia with Lewy bodies, and/or psychiatric and developmental disorders, including obsessive-compulsive disorder, Tourette's syndrome, depression, schizophrenia , attention-deficit/hyperactivity disorder, and autism (asperger).

2. The composition for use according to claim 1 wherein executive function involves speed of information processing, cognitive and mental flexibility, attention, scanning, and/or cognitive set shifting,

3. The composition for use according to any one of the preceding claims, wherein said subject suffers from Alzheimer's Disease or dementia syndrome, including mild or prodromal AD or dementia.

4. The composition for use according to any one of the preceding claims, wherein said subject has a mini-mental state examination (MMSE) of 20 - 30, preferably 20 - 26, more preferably 24 - 26.

5. The composition for use according to any one of the preceding claims, wherein said composition comprises choline, or salts or esters thereof, preferably 200 - 600 mg choline per daily dose or per 100 ml composition.

6. The composition for use according to any one of the preceding claims, wherein said composition comprises at least one, preferably at least two, most preferably all B vitamins selected from the group consisting of vitamin B6, vitamin B12 and vitamin B9.

7. The composition for use according to any one of the preceding claims, wherein said composition comprises, per daily dose or preferably per 100 ml composition, at least 500 mg of DHA, preferably at least 600 mg of DHA, and at least 50 mg of uridine, preferably at least 100 mg of uridine.

8. The composition for use according to any one of the preceding claims, wherein the composition comprises, per daily dose or preferably per 100 ml composition:
50-1000 mg phospholipids,
0.5-3 mg vitamin B6,
50-500 µg folic acid,
1-30 µg vitamin B12.

9. The composition for use according to any one of the preceding claims, wherein the composition comprises, per daily dose or preferably per 100 ml composition:
100 - 500 mg, preferably 200-400 mg EPA,
1000 - 1500 mg, preferably 1100-1300 mg DHA,
50 - 600 mg, preferably 60-200 mg phospholipids,
200 - 600 mg, preferably 300-500 mg choline,
400 - 800 mg, preferably 500-700 mg UMP (uridine monophosphate),
20 - 60 mg, preferably 30-50 mg vitamin E (alpha-TE),
60 - 100 mg, preferably 70-90 mg vitamin C,
40 - 80 µg, preferably 50-70 µg selenium,
1 - 5 µg, preferably 2-4 µg vitamin B12,
0.5 - 3 mg, preferably 0.5-2 mg vitamin B6, and
200 - 600 µg, preferably 300-500 µg folic acid.

## Patentansprüche

1. Eine Zusammensetzung zur Verwendung bei der Verbesserung der Ausführungsfunktion (,executive function') eines Subjekts, das dessen bedarf, wobei die Zusammensetzung umfasst:
i) eines oder mehrere von Uridin und Cytidin, oder Salze, Phosphate, Acylderivate oder Ester davon; und
ii) eine Lipidfraktion, die wenigstens eine von Docosahexaensäure (22:6; DHA), Eicosapentaensäure (20:5; EPA) und Docosapentaensäure (22:5; DPA) oder deren Ester umfasst,
wobei das Subjekt unter einer Gedächtnis- oder einer kognitiven Störung, einem Gedächtnisabnahme oder einer kognitiven Funktionsstörung leidet, wie beispielsweise Altersbedingte Gedächtnisstörungen (AAMI), Alzheimer-Krankheit, Multiple Sklerose, vaskuläre Demenz, frontotemporale Demenz, semantische Demenz oder Demenz mit Lewy-Körpern und/oder psychiatrische und Entwicklungsstörungen, einschließlich Zwangsstörungen, Tourette-Syndrom, Depression, Schizophrenie, Aufmerksamkeitsdefizit-/Hyperaktivitätsstörung und Autismus (Asperger).

2. Die Zusammensetzung zur Verwendung nach Anspruch 1, wobei die Ausführungsfunktion mit der Geschwindigkeit der Informationsverarbeitung, kognitiven und mentalen Flexibilität, Aufmerksamkeit, Abtasten und/oder Aufmerksamkeit-Set-Shifting verbunden ist.

3. Die Zusammensetzung zur Verwendung nach einem der vorhergehenden Ansprüche, wobei der Patient an Alzheimer-Krankheit oder Demenz-Syndrom leidet, einschließlich milder oder prodromaler AD oder Demenz.

4. Die Zusammensetzung zur Verwendung nach einem der vorhergehenden Ansprüche, wobei das Subjekt einen Mini-Mental-Status-Test (MMST) von 20 - 30, vorzugsweise 20 - 26, weiter bevorzugt 24 - 26, aufweist.

5. Die Zusammensetzung zur Verwendung nach einem der vorhergehenden Ansprüche, wobei die Zusammensetzung Cholin oder Salze oder Ester davon umfasst, vorzugsweise 200 - 600 mg Cholin pro Tagesdosis oder pro 100 ml Zusammensetzung.

6. Die Zusammensetzung zur Verwendung nach einem der vorhergehenden Ansprüche, wobei die Zusammensetzung mindestens ein, vorzugsweise mindestens zwei, am meisten bevorzugt alle B-Vitamine umfasst, die ausgewählt sind aus der Gruppe bestehend aus Vitamin B6, Vitamin B12 und Vitamin B9.

7. Die Zusammensetzung zur Verwendung nach einem der vorhergehenden Ansprüche, wobei die Zusammensetzung pro Tagesdosis oder vorzugsweise pro 100 ml Zusammensetzung mindestens 500 mg DHA, vorzugsweise mindestens 600 mg DHA, und mindestens 50 mg Uridin, vorzugsweise mindestens 100 mg Uridin, umfasst.

8. Die Zusammensetzung zur Verwendung nach einem der vorhergehenden Ansprüche, wobei die Zusammensetzung pro Tagesdosis oder vorzugsweise pro 100 ml Zusammensetzung umfasst:
50 - 1000 mg Phospholipide,
0,5 - 3 mg Vitamin B6,
50 - 500 µg Folsäure,
1 - 30 µg Vitamin B12.

9. Die Zusammensetzung zur Verwendung nach einem der vorhergehenden Ansprüche, wobei die Zusammensetzung pro Tagesdosis oder vorzugsweise pro 100 ml Zusammensetzung umfasst:
100 - 500 mg, vorzugsweise 200 - 400 mg EPA,
1000 - 1500 mg, vorzugsweise 1100 - 1300 mg DHA,
50 - 600 mg, vorzugsweise 60 - 200 mg Phospholipide,
200 - 600 mg, vorzugsweise 300 - 500 mg Cholin,
400 - 800 mg, vorzugsweise 500 - 700 mg UMP (Uridinmonophosphat),
20 - 60 mg, vorzugsweise 30 - 50 mg Vitamin E (alpha-TE),
60 - 100 mg, vorzugsweise 70 - 90 mg Vitamin C,
40 - 80 µg, vorzugsweise 50 - 70 µg Selen,
1 - 5 µg, vorzugsweise 2 - 4 µg Vitamin B12,
0,5 - 3 mg, vorzugsweise 0,5 - 2 mg Vitamin B6, und
200 - 600 µg, vorzugsweise 300 - 500 µg Folsäure.

## Revendications

1. Composition à utiliser pour améliorer la fonction exécutive d'un sujet qui en a besoin, dans laquelle ladite composition comprend :
i) un ou plusieurs parmi de l'uridine et de la cytidine, ou des sels, phosphates, dérivés acylés ou esters de celles-ci ; et
ii) une fraction lipidique comprenant au moins un parmi l'acide docosahexaénoïque (22 : 6 ; DHA), l'acide eicosapentaénoïque (20 : 5 ; EPA) et l'acide docosapentaénoïque (22 : 5 ; DPA), ou des esters de ceux-ci,
dans lequel ledit sujet souffre d'un trouble de la mémoire ou cognitif, d'un déclin de la mémoire ou d'un dysfonctionnement cognitif, tel qu'un trouble de la mémoire associé à l'âge (AAMI), de la maladie d'Alzheimer, de la sclérose en plaques, de la démence vasculaire, de la démence fronto-temporale, de la démence sémantique ou de la démence à corps de Lewy, de troubles psychiatriques et du développement, y compris d'un trouble obsessionnel-compulsif, du syndrome de Gilles de La Tourette, de dépression, de schizophrénie, d'un trouble de déficit d'attention/d'hyperactivité et d'autisme (asperger).

2. Composition à utiliser selon la revendication 1, dans laquelle la fonction exécutive implique une vitesse de traitement d'information, une flexibilité cognitive et mentale, une attention, un balayage et/ou un décalage cognitif.

3. Composition à utiliser selon l'une quelconque des revendications précédentes, dans laquelle ledit sujet souffre de la maladie d'Alzheimer ou d'un syndrome de démence, y compris une AD ou démence légère ou prodromique.

4. Composition à utiliser selon l'une quelconque des revendications précédentes, dans laquelle ledit sujet présente un mini-examen de l'état mental (MMSE) de 20 à 30, de préférence de 20 à 26 et de manière plus préférée de 24 à 26.

5. Composition à utiliser selon l'une quelconque des revendications précédentes, dans laquelle ladite composition comprend de la choline, ou des sels ou esters de celle-ci, de préférence 200 à 600 mg de choline par dose quotidienne ou par 100 ml de composition.

6. Composition à utiliser selon l'une quelconque des revendications précédentes, dans laquelle ladite composition comprend au moins une, de préférence au moins deux, de la manière la plus préférée toutes les vitamines B sélectionnées dans le groupe constitué de la vitamine B6, de la vitamine B12 et de la vitamine B9.

7. Composition à utiliser selon l'une quelconque des revendications précédentes, dans laquelle ladite composition comprend, par dose quotidienne ou de préférence par 100 ml de composition, au moins 500 mg de DHA, de préférence au moins 600 mg de DHA, et au moins 50 mg d'uridine, de préférence au moins 100 mg d'uridine.

8. Composition à utiliser selon l'une quelconque des revendications précédentes, dans laquelle la composition comprend, par dose journalière ou de préférence par 100 ml de composition :
50 à 1000 mg de phospholipides,
0,5 à 3 mg de vitamine B6,
50 à 500 µg d'acide folique,
1 à 30 µg de vitamine B12.

9. Composition à utiliser selon l'une quelconque des revendications précédentes, dans laquelle la composition comprend par dose journalière ou de préférence par 100 ml de composition :
100 à 500 mg, de préférence 200 à 400 mg d'EPA,
1000 à 1500 mg, de préférence 1100 à 1300 mg de DHA,
50 à 600 mg, de préférence 60 à 200 mg de phospholipides,
200 à 600 mg, de préférence 300 à 500 mg de choline,
400 à 800 mg, de préférence 500 à 700 mg d'UMP (monophosphate d'uridine),
20 à 60 mg, de préférence 30 à 50 mg de vitamine E (alpha-TE),
60 à 100 mg, de préférence 70 à 90 mg de vitamine C,
40 à 80 µg, de préférence 50 à 70 µg de sélénium,
1 à 5 µg, de préférence 2 à 4 µg de vitamine B12,
0,5 à 3 mg, de préférence 0,5 à 2 mg de vitamine B6, et
200 à 600 µg, de préférence 300 à 500 µg d'acide folique.
